(19) Europäisches Patentamt
European Patent Office
Office européen des brevets

(11) Veröffentlichungsnummer: **0 274 033 B1**

(12) # EUROPÄISCHE PATENTSCHRIFT

(45) Veröffentlichungstag der Patentschrift: **11.03.92**

(51) Int. Cl.$^5$: **C07D 215/56**, C07D 471/04, C07D 401/04, //(C07D471/04, 221:00,221:00)

(21) Anmeldenummer: **87117130.2**

(22) Anmeldetag: **20.11.87**

Die Akte enthält technische Angaben, die nach dem Eingang der Anmeldung eingereicht wurden und die nicht in dieser Patentschrift enthalten sind.

(54) **Verfahren zur Herstellung von Chinolincarbonsäuren.**

(30) Priorität: **03.12.86 DE 3641312**

(43) Veröffentlichungstag der Anmeldung:
**13.07.88 Patentblatt 88/28**

(45) Bekanntmachung des Hinweises auf die Patenterteilung:
**11.03.92 Patentblatt 92/11**

(84) Benannte Vertragsstaaten:
**AT BE CH DE ES FR GB GR IT LI LU NL SE**

(56) Entgegenhaltungen:
**EP-A- 0 078 362**
**EP-A- 0 126 355**
**EP-A- 0 167 763**
**EP-A- 0 195 841**
**DE-A- 2 656 574**
**DE-B- 2 804 097**

**PATENT ABSTRACTS OF JAPAN, Band 10, Nr. 178 (C-355)[2234], 21. Juni 1986; & JP - A - 61 24572 (KANEBO LTD.) 03.02.1986**

**PATENT ABSTRACTS OF JAPAN, Band 4, Nr. 85 (C-15)[567], 18. Juni 1980; & JP - A - 55 47658 (KIYOURIN SEIYAKU K.K.) 04.04.1980**

(73) Patentinhaber: **BAYER AG**
**W-5090 Leverkusen 1 Bayerwerk(DE)**

(72) Erfinder: **Preiss, Michael, Dr.**
**Egenstrasse 21**
**W-5600 Wuppertal 1(DE)**

EP 0 274 033 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein neues Verfahren zur Herstellung von Chinolincarbonsäuren, die als solche oder in Form ihrer Ester, Salze, Prodrugs usw. als antibakterielle Mittel in der Human- und Tiermedizin sowie als die Immunabwehr stimulierende Mittel verwendet werden.

Aus der DE-A 28 04 097, EP-A-0 195 841, US-P 4 448 962, DE-A 26 56 574, EP-A-0 078 362, EP-A-0 167 763 sowie EP-A-0 126 355 sind bereits Verfahren zur Herstellung von Chinoloncarbonsäurederivaten, die in 7-Position mit cyclischen Aminen substituiert sind, bekanntgeworden. Bei diesen Verfahren werden die cyclischen Amine jedoch nicht in reiner Form, d.h. ohne Lösungsmittel und Hydratwasser eingesetzt. Die nach diesen Verfahren erzielten Ausbeuten und Produktreinheiten sind nicht zufriedenstellend. Überraschend wurde nun gefunden, daß der völlige Verzicht auf zusätzliche Lösungsmittel und die Verwendung des reinen hydrat- oder kristallwasserfreien Amins verschiedene Vorteile mit sich bringt. Gegenstand der Erfindung ist daher ein Verfahren zur Herstellung von Verbindungen der Formel (I)

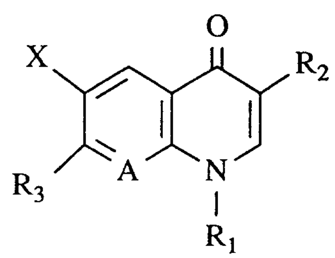

(I)

in welcher

- $R^1$ für Methyl, Ethyl, Propyl, Isopropyl, Cyclopropyl, 2-Hydroxyethyl, 2-Fluorethyl, 4-Fluorphenyl, 2,4-Difluorphenyl,
- $R^2$ für CN oder $COOR^{2'}$ mit $R^{2'}$ in der Bedeutung von Wasserstoff oder $C_1$-$C_4$-Alkyl,
- $R^3$ für eine cyclische Aminogruppe wie

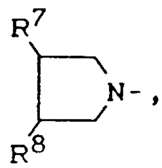 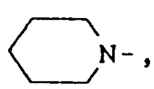 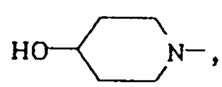 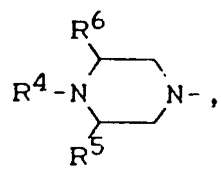

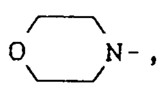 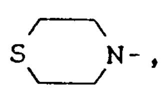 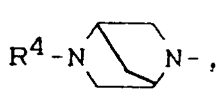 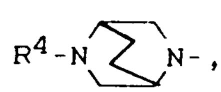

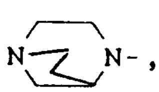 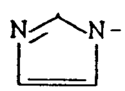 , 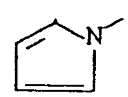

steht, worin

- $R^4$ für Wasserstoff, Alkyl mit 1 bis 4 Kohlenstoffatomen, 2-Hydroxyethyl, 2-Oxopropyl,
- $R^5$ für Wasserstoff oder Methyl,
- $R^6$ für Wasserstoff, Alkyl mit 1 bis 4 Kohlenstoffatomen,
- $R^7$ für Wasserstoff, Amino, Methylamino, Ethylamino, Aminomethyl, Methylaminomethyl, Ethylaminomethyl, Dimethylaminomethyl, Hydroxy, Hydroxymethyl,
- $R^8$ für Wasserstoff, Methyl, Ethyl oder Chlor steht,
- X für Halogen, insbesondere Fluor und Chlor oder
- A für C-$R^{11}$ steht, worin $R^{11}$ für Wasserstoff oder Halogen steht,

das dadurch gekennzeichnet ist, daß man Verbindungen der Formel (II)

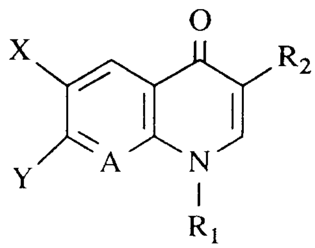

(II)

worin

$R^1$, $R^2$, A und X die oben angegebene Bedeutung haben, und
Y für Halogen, insbesondere Fluor und Chlor steht,

mit lösungsmittelfreien Aminen der Formeln

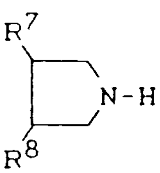, 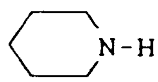, 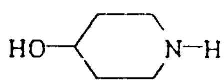, 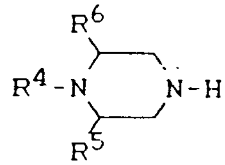,

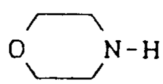, 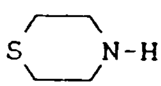, 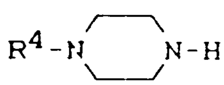, 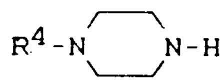,

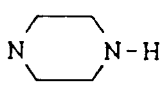, 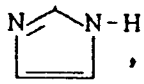, 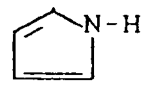.

worin

$R^4$, $R^5$, $R^6$, $R^7$, $R^8$ und $R^{11}$ die oben angegebene Bedeutung haben,

unter Verzicht auf die Anwendung von Lösungsmitteln bei Temperaturen 20 °C und 200 °C umsetzt.

Die genannten Amine sind bei dem erfindungsgemäßen Verfahren Reaktand und Lösungsmittel zugleich. Sind die Amine bei Raumtemperatur fest, werden sie aufgeschmolzen, die Reaktion mit (11) wird dann in der Schmelze durchgeführt.

Es ist als ausgesprochen überraschend zu bezeichnen, daß

a) die Reaktion von (II) mit den reinen Aminen mit sehr viel höherer Geschwindigkeit abläuft als bei Verwendung eines zusätzlichen Lösungsmittels, daß

b) die Reaktion bei tieferen Temperaturen abläuft, wodurch weniger Nebenprodukte gebildet werden und daß

c) trotz des Überschusses an Amin keine vermehrte Substitution von X stattfindet.

Letzterer Befund ist umso überraschender, weil nämlich, wenn (II) in das Amin eindosiert wird, zumindest am Anfang ein nahezu unendlich großer Überschuß an Amin vorliegt.

Die Vorteil des erfindungsgemäßen Verfahrens sind demnach:

- Reineres Produkt (weniger Nebenprodukte) durch die niedrigere Reaktionstemperatur
- Zeitersparnis durch die höhere Reaktionsgeschwindigkeit
- Ökonomischer Vorteil durch Wegfall eines zusätzlichen Lösungsmittels.

Das Verhältnis der Verbindungen (II) und der Amine kann 1:2 bis 1:10 betragen, bevorzugt 1:3 bis 1:5. Die Reaktionstemperatur liegt bei 20 °C bis 200 °C, bevorzugt wird der Bereich von 80 °C bis 180 °C, ganz besonders bevorzugt 120 °C bis 160 °C.

Bevorzugt werden in dem erfindungsgemäßen Verfahren folgende Amine eingesetzt:

Cyclische Amine wie Morpholin, Piperidin, Thiomorpholin, Pyrrolidin, Piperazin, N-Methylpiperazin, N-Ethylpiperazin, N-(2-Hydroxyethyl)-piperazin, 2-Methylpiperazin, 1,2-Dimethylpiperazin, cis- und trans-2,5-Dimethylpiperazin, cis- und trans-2,6-Dimethylpiperazin, 2-Ethylpiperazin, 2-Propylpiperazin, 2-Isopropylpiperazin und 2-Isobutylpiperazin.

Die Chinolincarbonsäuren der Formel (II) werden wie im folgenden beschrieben hergestellt:

Beispiel A

6-Chlor-1-cyclopropyl-7,8-difluor-1,4-dihydro-4-oxo-3-chinolincarbonsäure

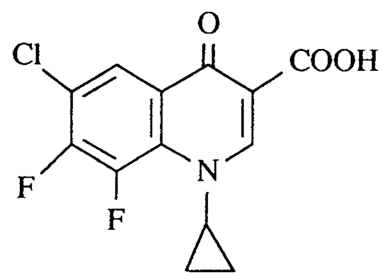

15,7 g (0,65 mol) Magnesiumspäne werden in 40 ml Ethanol und 2 ml Tetrachlormethan verrührt und nach begonnener Reaktion bei 50 bis 60° tropfenweise mit 103 g (0,64 mol) Malonsäurediethylester in 80 ml Ethanol und 250 ml Toluol versetzt. Man rührt 1 Stunde bei dieser Temperatur nach, kühlt auf -5 bis -10°, tropft eine Lösung von 138 g (0,65 mol) 5-Chlor-2,3,4-trifluorbenzoylfluorid in 63 ml Toluol zu, rührt noch 1 Stunde bei 0° und läßt über Nacht bei Raumtemperatur stehen. Danach wird noch 2 Stunden auf 40 bis 50° erwärmt, abgekühlt und mit 250 ml Eiswasser und 38,5 ml konzentrierter Schwefelsäure versetzt. Die organische Phase wird abgetrennt, die wäßrige Phase mit 2 mal 150 ml Toluol extrahiert, die vereinigten organischen Phasen mit gesättigter Natriumchlorid-Lösung gewaschen, mit Natriumsulfat getrocknet und eingeengt.

Der Rückstand wird mit 200 ml Wasser versetzt (vorteilhaft ist hier die Zugabe von 0,4 g 4-Toluolsulfonsäure) und zur Desethoxycarboxylierung-5 Stunden unter Rückfluß erhitzt. Man extrahiert mit 3 mal 200 ml Dichlormethan, wäscht mit gesättigter Natriumchlorid-Lösung, trocknet mit Natriumsulfat, engt ein und destilliert im Hochvakuum. Man erhält 103 g (56,5 %) (5-Chlor-2,3,4-trifluor-benzoyl)-essigsäure-ethylester mit einem Siedepunkt von 110°/0,9 Torr.

103 g (0,37 mol) des erhaltenen Esters und 83 g (0,56 mol) Orthoameisensäuretriethylester werden mit 95 g Essigsäureanhydrid 2 Stunden auf 150 bis 160° erhitzt und anschließend bei 120 bis 130° unter Normaldruck, danach im Hochvakuum eingeengt. Man erhält 115 g (92 % der Theorie) 2-(5-Chlor-2,3,4-trifluor-benzoyl)-3-ethoxy-acrylsäure-ethylester als Öl.

84,1 g (0,25 mol) dieser Verbindung werden in 170 ml Ethanol unter Eiskühlung tropfenweise mit 14,8 g (0,26 mol) Cyclopropylamin versetzt und 2 Stunden bei Raumtemperatur gerührt. Danach wird mit 170 ml Wasser verrührt, in Eis gekühlt, der ausgefallene Niederschlag abgesaugt, mit Wasser und wenig Methanol gewaschen und getrocknet. Es werden 47 g (54 %) 2-(5-Chlor-2,3,4-trifluor-benzoyl)-3-cyclopropylamino-acrylsäureethylester vom Schmelzpunkt 71 bis 73° erhalten. Nach dem $^1$H-NMR-Spektrum liegt ein cis-trans-Gemisch vor.

47 g (0,14 mol) dieser Verbindung werden in 230 ml Dimethylformamid mit 9,7 g (0,23 mol) Natriumfluorid 2 Stunden auf 160 bis 170° erhitzt, Das Reaktionsgemisch wird in 400 ml Eiswasser eingegossen, der Niederschlag abgesaugt, mit Wasser gewaschen und getrocknet. Man isoliert 44 g (99 %) 6-Chlor-1-cyclopropyl-7,8-difluor1,4-dihydro-4-oxo-3-chinolincarbonsäure-ethylester vom Schmelzpunkt 169 bis 172°.

44 g (0,13 mol) des Chinoloncarbonsäureesters werden in 300 ml Eisessig und 179 ml Wasser mit 33 ml konzentrierter Schwefelsäure versetzt und 2 Stunden auf 150°C erhitzt. Das Reaktionsgemisch wird in 400 ml Eiswasser eingerührt, der Niederschlag abgesaugt, mit Wasser gewaschen und getrocknet. Es werden 37 g (95 % der Theorie) 6-Chlor-1-cyclopropyl-7,8-difluor-1,4-dihydro-4-oxo-3-chinolincarbonsäure mit einem Schmelzpunkt von 200 bis 204° isoliert.

Beispiel B

8-Chlor-1-cyclopropyl-6,7-difluor-1,4-dihydro-4-oxo-3-chinolincarbonsäure

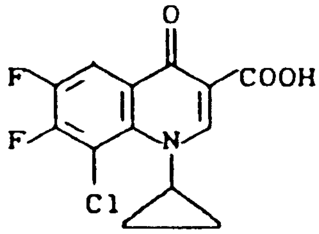

Man setzt 3-Chlor-2,4,5-trifluor-benzoylchlorid analog Beispiel A um, wobei folgende Stufen durchlaufen werden:
(3-Chlor-2,4,5-trifluor-benzoyl)-essigsäure-ethylester als Enol (Ausbeute: 42 %, Schmelzpunkt 72-75°),
2-(3-Chlor-2,4,5-trifluor-benzoyl-3-ethoxy-acrylsäureethylester (Rohausbeute: 95 % Öl),
2-(3-Chlor-2,4,5-trifluor-benzyl)-3-cyclopropyl-amino-acrylsäureethylester (Ausbeute: 67 %, Schmelzpunkt 78-80°),
8-Chlor-1-cyclopropyl-6,7-difluor-1,4-dihydro-4-oxo-3-chinolincarbonsäure-ethylester (Ausbeute: 85 %, Schmelzpunkt 154-157°),
8-Chlor-1-cyclopropyl-6,7-difluor-1,4-dihydro-4-oxo-3-chinolincarbonsäure (Ausbeute: 97,6 %, Schmelzpunkt 189-192°).

Beispiel C

6,8-Dichlor-1-cyclopropyl-7-fluor-1,4-dihydro-4-oxo-3-chinolincarbonsäure

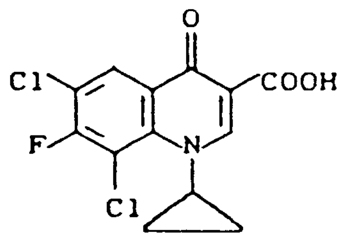

Man setzt 3,5-Dichlor-2,4-difluor-benzoylfluorid analog Beispiel A um, wobei folgende Stufen durchlaufen werden:
(3,5-Dichlor-2,4-difluor-benzoyl)-essigsäure-ethylester (Ausbeute: 43 %, Siedepunkt 133°/2,5 Torr),
2-(3,5-Dichlor-2,4-difluor-benzoyl)-3-ethoxy-acrylsäure-ethylester(Rohausbeute: 91 % Öl),
2-(3,5-Dichlor-2,4-difluor-benzoyl)-3-cyclopropyl-amino-acrylsäureethylester (Ausbeute: 96 %, Schmelzpunkt 71-74°),
6,8-Dichlor-1-cyclopropyl-7-fluor-1,4-dihydro-4-oxo-3-chinolincarbonsäure-ethylester (Ausbeute: 97 %, Schmelzpunkt 215-217° unter Zersetzung),
6,8-Dichlor-1-cyclopropyl-7-fluor-1,4-dihydro-4-oxo-4-chinolincarbonsäure (Ausbeute: 93 %, Schmelzpunkt 204-206°).

Weitere Chinolincarbonsäuren, insbesondere Cyclopropyl-6,7,8-trifluor-1,4-dihydro-4-oxo-3-chinolincarbonsäuren können gemäß folgendem Reaktionsschema hergestellt werden:

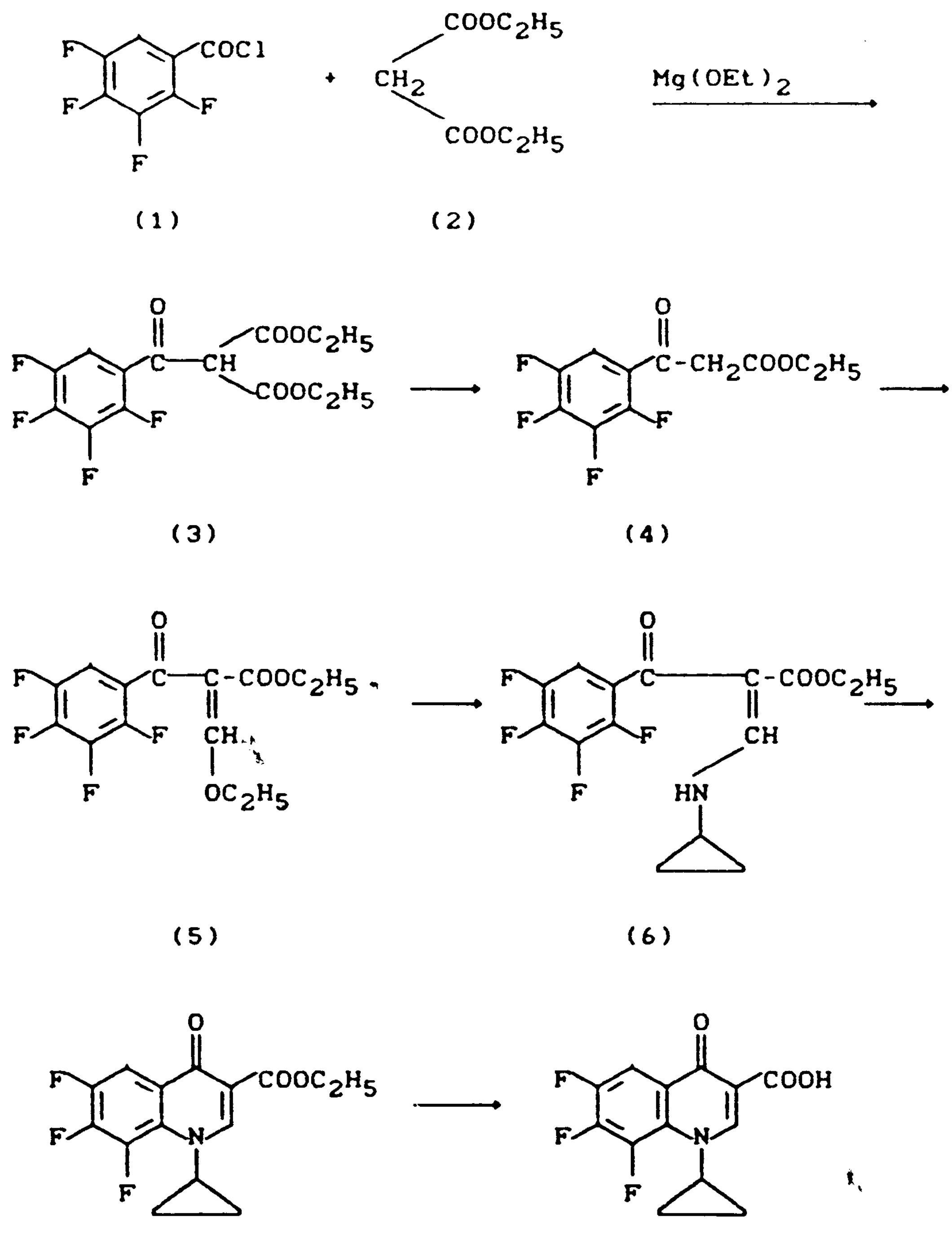


(1) (2)

(3) (4)

(5) (6)

(7) (II)

Danach wird Malonsäurediethylester (2) in Gegenwart von Magnesiumethylat mit 2,3,4,5-Tetrafluorbenzoylchlorid (1) zum Aroylmalonester (3) acyliert (Organicum, 3. Aufl. 1964, S. 438).

An Stelle von (1) kann auch das 2,3,4,5-Tetrafluorbenzoesäurefluorid verwendet werden.

Durch partielle Verseifung und Decarboxylierung von (3) in wäßrigem Medium mit katalytischen Mengen Schwefelsäure oder p-Toluolsulfonsäure erhält man in guter Ausbeute den Aroylessigsäureethylester (4), der mit o-Ameisensäure-triethylester/Acetanhydrid in den 2-(2,3,4,5-Tetrafluorbenzoyl)-3-ethoxy-acrylsäureethylester (5) übergeht. Die Umsetzung von (5) mit Cyclopropylamin in einem Lösungsmittel, wie z.B. Methylenchlorid, Alkohol, Chloroform, Cyclohexan oder Toluol führt in leicht exothermer Reaktion zum gewünschten Zwischenprodukt (6).

Die Cyclisierungsreaktion (6) → (7) wird in einem Temperaturbereich von etwa 60 bis 300 ° C, bevorzugt

80 bis 180 ° C durchgeführt.

Als Verdünnungsmittel können Dioxan, Dimethylsulfoxid, N-Methylpyrrolidon, Sulfolan, Hexamethylphosphorsäuretriamid und bovorzugt N,N-Dimethylformamid verwendet werden.

Als Säurebinder kommen für diese Reaktionsstufe Kaliumtert.-butanolat, Butyl-lithium, Lithium-phenyl, Phenyl-magnesiumbromid, Natriummethylat, Natriumhydrid, Natrium- oder Kalium-carbonat und besonders bevorzugt Kalium- oder Natrium-fluorid in Betracht. Es kann vorteilhaft sein, einen Überschuß von 10 Mol-% an Base einzusetzen.

Die in dem letzten Schritt erfolgende Esterhydrolyse von (7) unter basischen oder sauren Bedingungen führt zu 1-Cyclopropyl-6,7,8-trifluor-1,4-dihydro-4-oxo-3-chinolincarbonsäure.

Das als Ausgangsmaterial für diesen Syntheseweg verwendete 2,3,4,5-Tetrafluorbenzoylchlorid (1) wurde aus der literaturbekannten 2,3,4,5-Tetrafluor-benzoesäure (G. G. Yakobson, V. N. Odinokov und N. N. Vorozhtsov Jr., Zh. Obsh. Khim. 36. 139 (1966) mit Thionylchlorid auf übliche Weise erhalten. Es besitzt einen Siedepunkt von 75-80 ° C/17 mbar. Das 2,3,4,5-Tetrafluorbenzoylfluorid besitzt einen Siedepunkt von 46 bis 47 ° C/20 mbar ($n_D^{20}$ : 1,4375).

Weitere Chinolincarbonsäuren können wie folgt hergestellt werden:

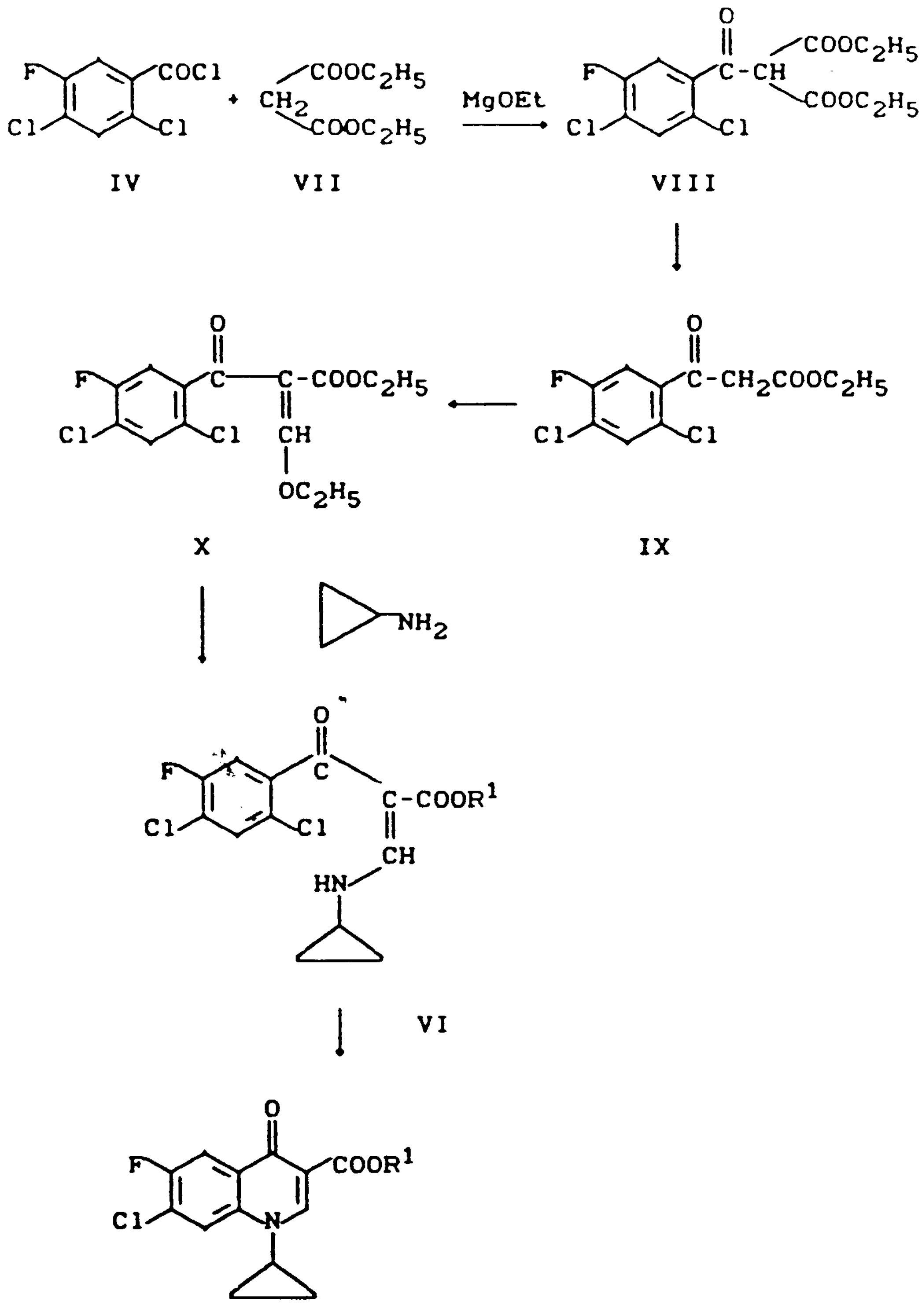


IV VII VIII

X IX

VI

II

Danach wird Malonsäurediethylester (VII) mit IV in Gegenwart von Magnesiumalkoholat zum Acylmalonester VIII acyliert (Organicum, 3. Aufl. 1964, S. 438).

Durch partielle Verseifung und Decarboxylierung von VIII in wäßrigem Medium mit katalytischen Mengen p-Toluolsulfonsäure erhält man in guter Ausbeute den Aroylessigsäureethylester IX, der mit o-Ameisensäuretriethylester/Acetanhydrid in den 2-(2,4-Dichlor-5-fluorbenzoyl)-3-ethoxy-acrylsäureethylester X übergeht. Die Umsetzung von X mit Cyclopropylamin in einem Lösungsmittel, wie z.B. Methylenchlorid, Alkohol, Chloroform, Cyclohexan oder Toluol führt in leicht exothermer Reaktion zum gewünschten Zwischenprodukt VI.

Die Cyclisierungsreaktion VI → II ($R^1$ = Alkyl) wird in einem Temperaturbereich von etwa 60° bis 280°C, bevorzugt 80° bis 180°C durchgeführt.

Als Verdünnungsmittel können Dioxan, Dimethylsulfoxid, N-Methyl-pyrrolidon, Sulfolan, Hexamethylphosphorsäuretriamid und bevorzugt N,N-Dimethylformamid verwendet werden.

Als Säurebinder kommen für diese Reaktionsstufe Kalium-t-butanolat, Butyllithium, Phenyllithium, Phenylmagnesiumbromid, Natriumethylat und besonders bevorzugt Natriumhydrid oder Kaliumcarbonat in Betracht. Es kann vorteilhaft sein, einen Überschuß von 10 Mol-% an Base einzusetzen.

Das als Ausgangsmaterial für diesen Syntheseweg verwendete 2,4-Dichlor-5-fluor-benzoylchlorid IV und die entsprechende Carbonsäure sowie das für die Herstellung von IV benötigte 3-Fluor-4,6-dichlortoluol XI sind aus EP-A2-0 078 362 bekannt.

Bevorzugte Verbindungen, die nach dem erfindungsgemäßen Verfahren hergestellt werden können sind in EP-A2-0 078 362, Seite 4, Zeilen 10 bis 16, EP-A-1 0049 355, Beispiele 1 bis 19, DE-OS 3 420 743, Seite 35, Zeile 20 bis Seite 37, Zeile 11 und DE-OS 3 318 145, Seite 31, Zeile 1 bis Seite 32, Zeile 13 beschrieben.

Weitere nach dem erfindungsgemäßen Verfahren herstellbare Verbindungen sind:
6-Chlor-1-cyclopropyl-7-(1,4-diazabicyclo[3.2.1]-oct-4-yl)-1,4-dihydro-4-oxo-3-chinolincarbonsäure,
1-Cyclopropyl-7-(1,4-diazabicyclo[3.2.1]-oct-4-yl)-6-fluor-1,4-dihydro-8-methyl-4-oxo-3-chinolincarbonsäure,
7-(1,4-Diazabicyclo[3.2.1]oct-4-yl)-6-fluor-1-(2-fluorethyl)-1,4-dihydro-4-oxo-3-chinolincarbonsäure,
7-(1,4-Diazabicyclo[3.2.1]oct-4-yl)-6-fluor-1-(4-fluorphenyl)-1,4-dihydro-4-oxo-3-chinolincarbonsäure,
1-Cyclopropyl-7-(1,4-diazabicyclo[3.2.1]oct-4-yl)-6-fluor-1,4-dihydro-4-oxo-1,8-naphthyridin-3-carbonsäure,
7-(1,4-Diazabicyclo[3.2.1]oct-4-yl)-1-ethyl-6-fluor-1,4-dihydro-4-oxo-1,8-naphthyridin-3-carbonsäure,
7-(1,4-Diazabicyclo[3.2.1]oct-4-yl)-1-ethyl-6-fluor-1,4-dihydro-4-oxo-3-chinolincarbonsäure,
7-(1,4-Diazabicyclo[3.2.1]oct-4-yl)-9-fluor-6,7-dihydro-5-methyl-1-oxo-1H,5H-benzo[i,j]chinolin-2-carbonsäure,
7-(1,4-Diazabicyclo[3.2.1]oct-4-yl)-6-fluor-1-(4-fluorphenyl)-1,4-dihydro-4-oxo-1,8-naphthyridin-3-carbonsäure,
1-Cyclopropyl-7-(1,4-diazabicyclo[3.2.1]oct-4-yl)-6-fluor-1,4-dihydro-4-oxo-3-chinolincarbonsäureethylester,
1-Cyclopropyl-7-(1,4-diazabicyclo[3.2.1]oct-4-yl)-6-fluor-1,4-dihydro-4-oxo-3-chinolincarbonsäure-(5-methyl-2-oxo-1,3-dioxol-4-yl-methyl)-ester,
1-Cyclopropyl-7-(1,4-diazabicyclo[3.1.1]hept-4-yl)-6-fluor-1,4-dihydro-4-oxo-3-chinolincarbonsäure,
1-Cyclopropyl-7-(1,4-diazabicyclo[3.1.1]hept-4-yl)-6,8-difluor-1,4-dihydro-4-oxo-3-chinolincarbonsäure,
8-Chlor-1-cyclopropyl-7-(1,4-diazabicyclo[3.1.1]hept-4-yl)-6-fluor-1,4-dihydro-4-oxo-3-chinolincarbonsäure,
1-Cyclopropyl-7-(1,4-diazabicyclo[3.1.1]hept-4-yl)-6-fluor-1,4-dihydro-8-methyl-4-oxo-3-chinolincarbonsäure,
1-Cyclopropyl-7-(1,4-diazabicyclo[3.1.1]hept-4-yl]-6-fluor-1,4-dihydro-4-oxo-1,8-naphthyridin-3-carbonsäure,
1-Cyclopropyl-7-(1,4-diazabicyclo[3.1.1]hept-4-yl)-6-fluor-1,4-dihydro-4-oxo-3-chinolincarbonsäuremethylester,
1-Cyclopropyl-6,8-difluor-1,4-dihydro-7-(8-methyl-3,8-diazabicyclo[3.2.1]oct-3-yl)-4-oxo-3-chinolincarbonsäure,
8-Chlor-1-cyclopropyl-6-fluor-1,4-dihydro-7-(8-methyl-3,8-diazabicyclo[3.2.1]oct-3-yl)-4-oxo-3-chinolincarbonsäure,
1-Cyclopropyl-6-fluor-1,4-dihydro-7-(8-methyl-3,8-diazabicyclo[3.2.1]oct-3-yl)-4-oxo-1,8-naphthyridin-3-carbonsäure,
1-Cyclopropyl-6-fluor-1,4-dihydro-7-(3-methyl-3,8-diazabicyclo[3.2.1]oct-8-yl)-4-oxo-3-chinolincarbonsäure,
1-Cyclopropyl-6,8-difluor-1,4-dihydro-7-(3-methyl-3,8-diazabicyclo[3.2.1]oct-8-yl)-4-oxo-3-chinolincarbonsäure,
8-Chlor-1-cyclopropyl-6-fluor-1,4-dihydro-7-(3-methyl-3,8-diazabicyclo[3.2.1]oct-8-yl)-4-oxo-3-chinolincarbonsäure,
1-Cyclopropyl-7-(2,5-diazabicyclo[2.2.1]hept-2-yl)-6-fluor-1,4-dihydro-4-oxo-3-chinolincarbonsäure,
1-Cyclopropyl-7-(2,5-diazabicyclo[2.2.1]hept-2-yl)-6,8-difluor-1,4-dihydro-4-axo-3-chinolincarbonsäure,
8-Chlor-1-cyclopropyl-7-(2,5-diazabicyclo[2.2.1]-hept-2-yl)-7-fluor-1,4-dihydro-4-oxo-3-chinolincarbonsäure,
1-Cyclopropyl-7-(2,5-diazabicyclo[2.2.1]hept-2-yl)-6-fluor-1,4-dihydro-4-oxo-1,8-naphthyridin-3-carbonsäure,
1-Cyclopropyl-6-fluor-1,4-dihydro-7-[5-(2-hydroxyethyl)-2,5-diazabicyclo[2.2.1]hept-2-yl]-4-oxo-3-chinolincarbonsäure,
1-Cyclopropyl-6-fluor-1,4-dihydro-4-oxo-7-[5-(2-oxo-propyl)-2,5-diazabicyclo[2.2.1]hept-2-yl]-3-chinolincarbonsäure,
1-Cyclopropyl-6-fluor-1,4-dihydro-7-(5-methyl-2,5-diazabicyclo[2.2.1]hept-2-yl)-4-oxo-3-chinolincarbonsäure,
1-Cyclopropyl-6,8-difluor-1,4-dihydro-7-(5-methyl-2,5-diazabicyclo[2.2.1]hept-2-yl)-4-oxo-3-chinolincarbonsäure,
8-Chlor-1-cyclopropyl-6-fluor-1,4-dihydro-7-(5-methyl-2,5-diazabicyclo[2.2.1]hept-2-yl)-4-oxo-3-chinolincarbonsäure,
1-Cyclopropyl-7-(8-ethyl-3,8-diazabicyclo[3.2.1]oct-3-yl)-6-fluor-1,4-dihydro-4-oxo-3-chinolincarbonsäure,
1-Cyclopropyl-6-fluor-1,4-dihydro-4-oxo-7-[8-(3-oxobutyl)-3,8-diazabicyclo[3.2.1]oct-3-yl]-3-chinolincarbonsäure,
7-[8-(4-Aminobenzyl)-3,8-diazabicyclo[3.2.1]oct-3-yl]-1-cyclopropyl-6-fluor-1,4-dihydro-4-oxo-3-chinolincarbonsäure,
1-Cyclopropyl-7-(2,5-diazabicyclo[2.2.2]oct-2-yl)-6-fluor-1,4-dihydro-4-oxo-3-chinolincarbonsäure,
1-Cyclopropyl-7-(2,5-diazabicyclo[2.2.2]oct-2-yl)-6,8-difluor-1,4-dihydro-4-oxo-3-chinolincarbonsäure,
8-Chlor-1-cyclopropyl-7-(2,5-diazabicyclo[2.2.2]oct-2-yl)-7-fluor-1,4-dihydro-4-oxo-3-chinolincarbonsäure,
1-Cyclopropyl-7-(2,5-diazabicyclo[2.2.2]oct-2-yl)-6-fluor-1,4-dihydro-4-oxo-1,8-naphthyridin-3-carbonsäure,
1-Cyclopropyl-6-fluor-1,4-dihydro-7-(5-methyl-2,5-diazabicyclo[2.2.2]oct-2-yl)-4-oxo-3-chinolincarbonsäure,
1-Cyclopropyl-6,8-difluor-1,4-dihydro-7-(5-methyl-2,5-diazabicyclo[2.2.2]oct-2-yl)-4-oxo-3-chinolincarbonsäure,
8-Chlor-1-cyclopropyl-6-fluor-1,4-dihydro-7-(5-methyl-2,5-diazabicyclo[2.2.2]oct-2-yl)-4-oxo-3-chinolincarbonsäure,
1-Cyclopropyl-7-(3,9-diazabicyclo[3.3.1]non-3-yl)-6-fluor-1,4-dihydro-4-oxo-3-chinolincarbonsäure,
1-Cyclopropyl-6-fluor-1,4-dihydro-7-(7-hydroxy-3,9-diazabicyclo[3.3.1]non-3-yl)-4-oxo-3-chinolincarbonsäure,
1-Cyclopropyl-6-fluor-1,4-dihydro-7-(3-oxa-7,9-diazabicyclo[3.3.1]non-7-yl)-4-oxo-3-chinolincarbonsäure,
7-(5-Allyl-2,5-diazabicyclo[2.2.1]hept-2-yl)-1-cyclopropyl-6-fluor-1,4-dlhydro-4-oxo-3-chinolincarbonsäure,
1-Cyclopropyl-6-fluor-1,4-dihydro-4-oxo-7-(5-propargyl-2,5-diazabicyclo[2.2.2]oct-2-yl)-3-chinolincarbonsäure.
1-Cyclopropyl-7-(1,4-diazabicyclo[3.2.1]oct-4-yl)-6-fluor-1,4-dihydro-4-oxo-3-chinolincarbonsäure-Hydrochlorid vom Schmelzpunkt 322 ° C,
1-Cyclopropyl-7-(1,4-diazabicyclo[3.2.1]oct-4-yl)-6-fluor-1,4-dihydro-4-oxo-3-chinolincarbonsäure-Hemiembonat vom Schmelzpunkt ab 271 ° C,
8-Chlor-1-cyclopropyl-7-(1,4-diazabicyclo[3.2.1]oct-4-yl)-6-fluor-1,4-dihydro-4-oxo-3-chinolincarbonsäure-Hydrochlorid-Hydrat vom Schmelzpunkt 310 ° C,
1-Cyclopropyl-7-(1,4-diazabicyclo[3.2.1]oct-4-yl)-6,8-difluor-1,4-dihydro-4-oxo-3-chinolincarbonsäure vom Schmelzpunkt 275-282 ° C,
1-Cyclopropyl-7-(1,4-diazabicyclo[3.2.1]oct-4-yl)-1,4-dihydro-6-nitro-4-oxo-3-chinolincarbonsäure-Hydrochlorid vom Schmelzpunkt 303-307 ° C,
1-Cyclopropyl-7-(1,4-diazabicyclo[3.2.1]oct-4-yl)-6-fluor-1,4-dihydro-4-oxo-1,8-naphthyridin-3-carbonsäure-Hydrochlorid vom Schmelzpunkt >300 ° C,
7-(1,4-Diazabicyclo[3.2.1]oct-4-yl)-1-ethyl-6,8-difluor-1,4-dihydro-4-oxo-3-chinolincarbonsäure-Hydrochlorid vom Schmelzpunkt 308-312 ° C,
10-(1,4-Diazabicyclo[3.2.1]oct-4-yl)-9-fluor-2,3-dihydro-3-methyl-7-oxo-7H-pyrido[1,2,3-de][1,4]benzoxacin-6-carbonsäure-Hydrochlorid vom Schmelzpunkt 355 ° C,
7-(1,4-Diazabicyclo[3.2.1]oct-4-yl)-6,8-difluor-1-(4-fluorphenyl)-1,4-dihydro-4-oxo-3-chinolincarbonsäure-Hydrochlorid vom Schmelzpunkt 310-314 ° C,
1-Cyclopropyl-7-(1,4-diazabicyclo[3.2.1]oct-4-yl)-6-fluor-1,4-dihydro-8-nitro-4-oxo-3-chinolincarbonsäure vom Schmelzpunkt 215-232 ° C.

Die hierfür benötigte 7-Chlor-1-cyclopropyl-6-fluor-1,4-dihydro-8-nitro-4-oxo-3-chinolincarbonsäure wird über folgende Stufen erhalten:

a) 2,4-Dichlor-5-fluor-3-nitro-benzoesäure

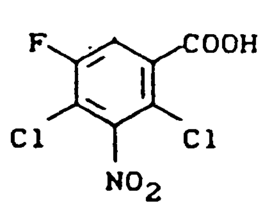

Unter Eiskühlung und Rühren werden 34 ml konzentrierter Schwefelsäure tropfenweise mit 40 ml konzentrierter Salpetersäure versetzt. In dieses Nitriergemisch trägt man portionsweise 20,9 g 2,4-Dichlor-5-fluorbenzoesäure ein, wobei die Temperatur auf 45 - 50 ° C steigt. Dann wird noch 3 Stunden auf 90 - 100 ° C erhitzt, das auf Raumtemperatur abgekühlte Gemisch auf 350 ml Eiswasser gegossen, der Niederschlag abgesaugt und mit Wasser gewaschen. Das feuchte Rohprodukt wird in 30 ml

Methanol heiß gelöst und die Lösung mit 150 ml Wasser versetzt.

Der Niederschlag wird kalt abgesaugt, mit Methanol/ Wasser gewaschen und im Vakuum bei 80 °C getrocknet. Es werden 21,2 g rohe 2,4-Dichlor-5-fluor-3-nitrobenzoesäure erhalten. Sie ist genügend rein für die weiteren Umsetzungen. Eine Probe aus Toluol/Petrolether umkristallisiert liefert Kristalle vom Schmelzpunkt 192 °C.

b) 2,4-Dichlor-5-fluor-3-nitro-benzoylchlorid

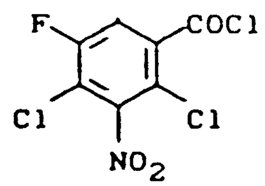

106,6 g 2,4-Dichlor-5-fluor-3-nitro-benzoesäure werden mit 250 ml Thionylchlorid 2 Stunden unter Rückfluß zum Sieden erhitzt. Das überschüssige Thionylchlorid wird dann bei Normaldruck abdestilliert und der Rückstand im Feinvakuum fraktioniert. Bei 110 -115 °C/0,08-0,09 mbar gehen 104,7 g 2,4-Dichlor-5-fluor-3-nitro-benzoylchlorid über. Beim Stehen bilden sich Kristalle vom Schmelzpunkt 35 bis 37 °C.

c) (2,4-Dichlor-5-fluor-3-nitro-benzoyl)-essigsäureethylester

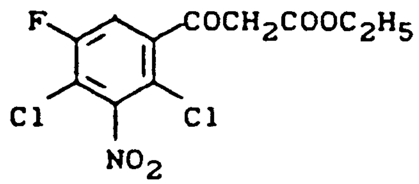

10,1 g Magnesiumspäne werden in 21 ml Ethanol mit 2,1 g Tetrachlormethan versetzt und nach Beginn der Wasserstoff-Entwicklung ein Gemisch aus 66,6 g Malonsäurediethylester, 40 ml Ethanol und 150 ml Toluol bei 50 - 60 °C tropfenweise zugefügt. Man rührt 1 Stunde bei dieser Temperatur nach, kühlt auf -5 bis -10 °C und tropft langsam eine Lösung von 109,2 g 2,4-Dichlor-5-fluor-3-nitro-benzoylchlorid in 50 ml Toluol zu. Danach wird 1 Stunde bei 0 °C gerührt, über Nacht auf Raumtemperatur gebracht und noch 2 Stunden auf 40 - 50 °C erwärmt. Das Reaktionsgemisch wird unter Eiskühlung mit einem Gemisch aus 160 ml Wasser und 10,4 ml konzentrierter Schwefelsäure versetzt und die organische Phase abgetrennt. Die wäßrige Phase wird mit Toluol extrahiert und der vereinigte organische Extrakt mit gesättigter Natriumchlorid-Lösung gewaschen, mit Natriumsulfat getrocknet und das Lösungsmittel abgezogen. Man erhält 144,5 g (2,4-Dichlor-5-fluor-3-nitro-benzoyl)-malonsäurediethylester als Rohprodukt. Dieses wird nach Zugabe von 200 ml Wasser und 0,6 g 4-Toluolsulfonsäure 3 Stunden unter Rückfluß erhitzt, die Mischung mit Methylenchlorid extrahiert, der Extrakt mit Natriumsulfat getrocknet und das Lösungsmittel im Vakuum abdestilliert. Es werden 118 g substituierter Benzoylessigester als Rohprodukt erhalten. Er besitzt eine für die weiteren Umsetzungen genügende Reinheit.

d) 7-Chlor-1-cyclopropyl-6-fluor-1,4-dihydro-8-nitro-4-oxo-3-chinolincarbonsäure

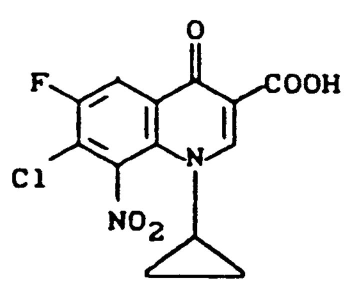

244,8 g (2,4-Dichlor-5-fluor-3-nitro-benzoyl)-essigsäureethylester werden mit 166 g Orthoameisensäuretriethylester und 185 g Essigsäureanhydrid 3 Stunden auf 150 -160 ° C erhitzt. Man engt im Vakuum ein und erhält 270 g 2-(2,4-Dichlor-5-fluor-3-nitro-benzoyl)-3-ethoxy-acrylsäure-ethylester als öligen Rückstand.

38 g dieser Zwischenstufe werden in 80 ml Ethanol unter Eiskühlung tropfenweise mit 5,9 g Cyclopropylamin versetzt und 1 Stunde bei 20 ° C gerührt.
Das ausgefallene Produkt wird nach Zugabe von 100 ml Wasser abgesaugt, mit Ethanol/$H_2O$ (1:1) gewaschen und getrocknet. Man erhält 32,8 g 2-(2,4-Dichlor-5-fluor-3-nitro-benzoyl)-3-cyclopropylamino-acrylsäureethylester vom Schmelzpunkt 143 - 146 ° C.

7,8 g der vorgenannten Verbindung werden in 30 ml wasserfreiem Dioxan mit 3,1 g 1,8-Diazabicyclo-[5.4.0]undec-7-en (DBU) versetzt und 4 Stunden auf 100 ° C erhitzt. Das Lösungsmittel wird im Vakuum abdestilliert, der Rückstand in Methylenchlorid/ Wasser aufgenommen, die Methylenchlorid-Phase abgetrennt, mit Natriumsulfat getrocknet und das Methylenchlorid abdestilliert. Es werden 7,2 g 7-Chlor-1-cyclopropyl-6-fluor-1,4-dihydro-8-nitro-4-oxo-3-chinolincarbonsäureethylester als Rohprodukt erhalten. Nach Umkristallisation aus Acetonitril schmelzen die hellbraunen Kristalle bei 174 - 175 ° C.
Ausbeute: 6 g.

7-(5-Benzyl-2,5-diazabicyclo[2.2.1]hept-2-yl)-1-cyclopropyl-6-fluor-1,4-dihydro-4-oxo-3-chinolincarbonsäure vom Schmelzpunkt 205 - 214 ° C.

1-Cyclopropyl-6-fluor-1,4-dihydro-7-(8-methyl-3,8-diazabicyclo[32.1]oct-3-yl)-4-oxo-3-chinolincarbonsäure vom Schmelzpunkt 273 - 278 ° C.

1-Cyclopropyl-6-fluor-1,4-dihydro-7-(5-methyl-1,4-diazabicyclo[3.2.1]oct-4-yl)-4-oxo-3-chinolincarbonsäure-Hydrochlorid vom Schmelzpunkt >300 ° C.

1,1 g 1-Cyclopropyl-7-(1,4-diazabicyclo[3.2.1]-oct-4-yl)-6,8-difluor-1,4-dihydro-4-oxo-3-chinolincarbonsäureethylester vom Schmelzpunkt 196-199 ° C.

7-(1,4-Diazabicyclo[3.2.1]oct-4-yl)-6,8-difluor-1-(2,4-difluorphenyl)-1,4-dihydro-4-oxo-3-chinolincarbonsäure-Hydrochlorid vom Schmelzpunkt 329-331 ° C.

7-(1,4-Diazabicyclo[3.2.1]oct-4-yl)-6,8-difluor-1,4-dihydro-1-methylamino-4-oxo-3-chinolincarbonsäure-Hydrochlorid vom Schmelzpunkt 300-305 ° C.

Weitere Wirkstoffe, die erfindungsgemäß hergestellt werden können, sind 6-Chlor-7-[3-(4-chlorphenyl)-1-piperazinyl)-1-cyclopropyl-8-fluor-1,4-dihydro-4-oxo-3-chinolincarbonsäure,
6-Chlor-1-cyclopropyl-8-fluor-7-[3-(4-fluorphenyl)-1-piperazinyl]-1,4-dihydro-4-oxo-3-chinolincarbonsäure,
7-[3-(4-Bromphenyl)-1-piperazinyl]-6-chlor-1-cyclopropyl-8-fluor-1,4-dihydro-4-oxo-3-chinolincarbonsäure,
6-Chlor-1-cyclopropyl-8-fluor-1,4-dihydro-8-[3-(4-methylphenyl)-1-piperazinyl]-4-oxo-3-chinolincarbonsäure,
7-[3-(4-Biphenylyl)-1-piperazinyl]-6-chlor-1-cyclopropyl-8-fluor-1,4-dihydro-4-oxo-3-chinolincarbonsäure,
6-Chlor-1-cyclopropyl-8-fluor-1,4-dihydro-7-[3-(4-methoxyphenyl)-1-piperazinyl]-4-oxo-3-chinolincarbonsäure,
6-Chlor-1-cyclopropyl-8-fluor-1,4-dihydro-7-[3-(4-hydroxyphenyl)-1-piperazinyl]-4-oxo-3-chinolincarbonsäure,
8-Chlor-1-cyclopropyl-6-fluor-1,4-dihydro-4-oxo-7-(3-phenyl-1-piperazinyl)-3-chinolincarbonsäure,
8-Chlor-1-cyclopropyl-6-fluor-1,4-dihydro-4-oxo-7-[(4-nitrophenyl)-1-piperazinyl]-3-chinolincarbonsäure,
8-Chlor-1-cyclopropyl-6-fluor-1,4-dihydro-4-oxo-7-[3-(4-piperidino-phenyl)-1-piperazinyl]-3-chinolincarbonsäure,
8-Chlor-1-cyclopropyl-6-fluor-1,4-dihydro-4-oxo-7-[3-(3,4-dimethoxy-phenyl)-1-piperazinyl]-3-chinolincarbonsäure,
8-Chlor-1-cyclopropyl-6-fluor-1,4-dihydro-4-oxo-7-[3-(3,4,5-trimethoxy-phenyl)-1-piperazinyl]-3-chinolincarbonsäure,
8-Chlor-1-cyclopropyl-6-fluor-1,4-dihydro-4-oxo-7-[3-(2-thienyl)-1-piperazinyl]-3-chinolincarbonsäure,
8-Chlor-1-cyclopropyl-6-fluor-1,4-dihydro-4-oxo-7-piperidino-3-chinolincarbonsäure,
7-(3-Amino-1-pyrrolidinyl)-8-chlor-1-cyclopropyl-6-fluor-1,4-dihydro-4-oxo-3-chinolincarbonsäure,
6,8-Dichlor-1-cyclopropyl-1,4-dihydro-4-oxo-7-(1-piperazinyl)-3-chinolincarbonsäure,
7-(4-Acetyl-1-piperazinyl)-6,8-dichlor-1-cyclopropyl-1,4-dihydro-4-oxo-3-chinolincarbonsäure,
7-(4-Acetyl-1-piperazinyl)-6-chlor-1-cyclopropyl-8-fluor-1,4-dihydro-4-oxo-3-chinolincarbonsäure,
6-Chlor-1-cyclopropyl-8-fluor-1,4-dihydro-7-(4-isopropyl-1-piperazinyl)-4-oxo-3-chinolincarbonsäure,
6-Chlor-1-cyclopropyl-8-fluor-1,4-dihydro-4-oxo-7-morpholino-3-chinolincarbonsäure,
6-Chlor-1-cyclopropyl-8-fluor-1,4-dihydro-4-oxo-7-thiomorpholino-3-chinolincarbonsäure,
8-Chlor-1-cyclopropyl-7-(4-ethyl-3-oxo-1-piperazinyl)-6-fluor-1,4-dihydro-4-oxo-3-chinolincarbonsäure.

Erfindungsgemäß werden in der Regel zunächst die freien Carbonsäuren erhalten, die danach in Salze, Ester, Prodrugs etc. nach bekannten Methoden überführt werden können.

Die ganz besonders bevorzugt nach dem erfindungsgemäßen Verfahren herstellbaren Verbindungen sind Ciprofloxacin und das entsprechende 1-Ethyl-piperazinderivat.

Beispiele

Beispiel 1

606 Gew.-Teile 1-Cyclopropyl-7-chlor-6-fluor-1,4-dihydro-4-oxo-3-chinolincarbonsäure und 575 Gew.-Teile Piperazin werden in einem geeigneten Reaktor 30 Minuten auf 150 bis 160 °C erhitzt. Das Reaktionsgemisch wird mit Wasser verdünnt, wobei in 71 %iger Ausbeute 1-Cyclopropyl-6-fluor-1,4-dihydro-4-oxo-7-(1-piperazinyl)-3-chinolincarbonsäure mit einem Gehalt von ca. 98 % erhalten wird.

Beispiel 2

133 Gew.-Teile 1-Cyclopropyl-6,7-difluor-1,4-dihydro-4-oxo-3-chinolincarbonsäure und 129 Gew.-Teile Piperazin werden wie in Beispiel 1 umgesetzt. Man erhält in 70 %iger Ausbeute 1-Cyclopropyl-6-fluor-1,4-dihydro-4-oxo-7-(1-piperazinyl)-3-chinolincarbonsäure mit einem Gehalt von 98,5 %.

Beispiel 3

143 Gew.-Teile Piperazin werden auf 140 bis 150 °C erhitzt. In diese Schmelze werden 94 Gew.-Teile 1-Cyclopropyl-7-chlor-6-fluor-1,4-dihydro-4-oxo-3-chinolincarbonsäure innerhalb von 20 Minuten eindosiert. Man kühlt auf 100 °C ab und gibt Wasser zu. Hierbei kristallisiert 1-Cyclopropyl-6-fluor-1,4-dihydro-4-oxo-7-(1-piperazinyl)-3-chinolincarbonsäure in 78 %iger Ausbeute mit einer Reinheit von 97,2 % aus.

Beispiel 4

150 Gew.-Teile Piperazin werden mit 102 Gew.-Teilen 1-Cyclopropyl-6,7-difluor-1,4-dihydro-4-oxo-3-chinolincarbonsäure wie in Beispiel 3 umgesetzt. Man erhält 1-Cyclopropyl-6-fluor-1,4-dihydro-4-oxo-7-(1-piperazinyl)-3-chinolincarbonsäure in einer Ausbeute von 76 % mit einer Reinheit von 97,5 %.

Beispiel 5

Zu 122 Gew -Teilen 1-Ethyl-piperazin, das auf 140 bis 150 °C erhitzt wurde; werden innerhalb von 30 Minuten 60 Gew.-Teile 1-Cyclopropyl-7-chlor-6-fluor-1,4-dihydro-4-oxo-3-chinolincarbonsäure gegeben. Nach Abkühlen auf 100 °C wird Wasser zugegeben, wobei 1-Cyclopropyl-6-fluor-1,4-dihydro-4-oxo-7-(4-ethyl-1-piperazinyl)-3-chinolincarbonsäure in 76 %iger Ausbeute und 98,2 %iger Reinheit auskristallisiert.

## Patentansprüche

**1.** Verfahren zur Herstellung von Verbindungen der Formel (I)

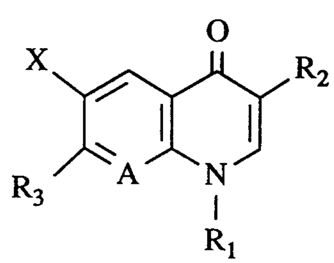

(I)

in welcher

$R^1$ für Methyl, Ethyl, Propyl, Isopropyl, Cyclopropyl, 2-Hydroxyethyl, 2-Fluorethyl, 4-Fluorphenyl, 2,4-Difluorphenyl,

$R^2$ für CN oder $COOR^{2'}$ mit $R^{2'}$ in der Bedeutung von Wasserstoff oder $C_1$-$C_4$-Alkyl,

$R^3$ für eine cyclische Aminogruppe wie

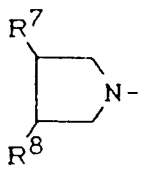, 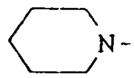, 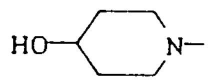, 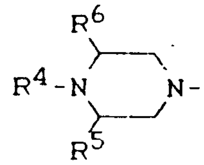,

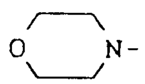, 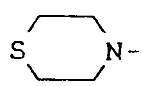, 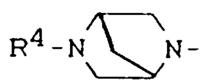, 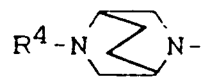,

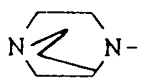, 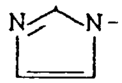, 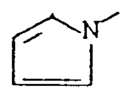

$R^4$ für Wasserstoff, Alkyl mit 1 bis 4 Kohlenstoffatomen, 2-Hydroxyethyl, 2-Oxopropyl,
$R^5$ für Wasserstoff oder Methyl,
$R^6$ für Wasserstoff, Alkyl mit 1 bis 4 Kohlenstoffatomen,
$R^7$ für Wasserstoff, Amino, Methylamino, Ethylamino, Aminomethyl, Methylaminomethyl, Ethylaminomethyl, Dimethylaminomethyl, Hydroxy, Hydroxymethyl,
$R^8$ für Wasserstoff, Methyl, Ethyl oder Chlor steht,
X für Halogen, insbesondere Fluor und Chlor oder
A für C-$R^{11}$ steht, worin $R^{11}$ für Wasserstoff oder Halogen steht,

dadurch gekennzeichnet ist, daß man Verbindungen der Formel (II)

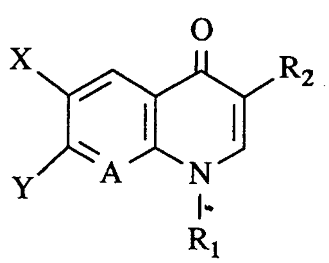 (II)

worin
$R^1$, $R^2$, A und X die oben angegebene Bedeutung haben, und
Y für Halogen, insbesondere Fluor und Chlor steht,

mit lösungsmittelfreien Aminen der Formeln

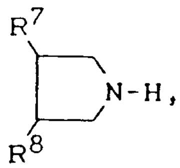 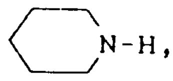 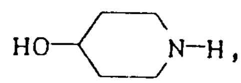 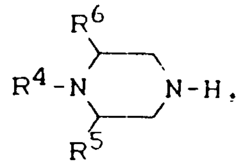

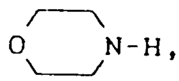 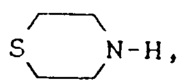 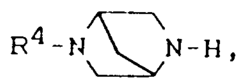 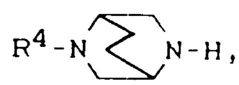

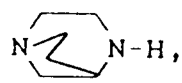 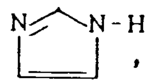 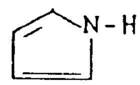

worin

$R^4$, $R^5$, $R^6$, $R^7$, $R^8$ und $R^{11}$ die oben angegebene Bedeutung haben,

unter Verzicht auf die Anwendung von Lösungsmitteln mit den reinen Aminen im Verhältnis 1:2 bis 1:10 bei Temperaturen zwischen 20 ° C und 200 ° C umsetzt.

**2.** Verfahren nach Anspruch 1, dadurch gekennzeichnet, daß man 1-Cyclopropyl-7-chlor-6-fluor-1,4-dihydro-4-oxo-3-chinolincarbonsäure lösungsmittelfrei mit Piperazin bei 120 ° bis 160 ° C umsetzt.

**3.** Verfahren nach den Ansprüchen 1 und 2, dadurch gekennzeichnet, daß man 1-Cyclopropyl-6,7-difluoro-1,4-dihydro-4-oxo-3-chinolincarbonsäure lösungsmittelfrei mit Piperazin bei 120 ° C bis 160 ° C umsetzt.

**4.** Verfahren nach den Ansprüchen 1 und 2, dadurch gekennzeichnet, daß man 1-Cyclopropyl-7-chlor-6-fluor-1,4-dihydro-4-oxo-3-chinolincarbonsäure in Piperazin, das auf 140 ° C bis 150 ° C erhitzt wurde, eindosiert, abkühlt, Wasser hinzufügt und das Umsetzungsprodukt auskristallisiert.

**5.** Verfahren nach Anspruch 4, dadurch gekennzeichnet, daß man 1-Cyclopropyl-6,7-difluoro-1,4-dihydro-4-oxo-3-chinolincarbonsäure als Ausgangsprodukt verwendet.

**6.** Verfahren nach Anspruch 1, dadurch gekennzeichnet, daß man 1-Cyclopropyl-7-chlor-6-fluor-1,4-dihydro-4-oxo-3-chinolincarbonsäure mit 1-Ethylpiperazin, 2-Hydroxyethylpiperazin oder 1-Methylpiperazin umsetzt.

## Claims

**1.** Process for the preparation of compounds of the formula (I)

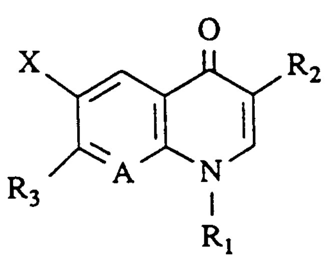

in which

$R^1$ represents methyl, ethyl, propyl, isopropyl, cyclopropyl, 2-hydroxyethyl, 2-fluoroethyl, 4-fluorophenyl or 2,4-difluorophenyl,

$R^2$ represents CN or $COOR^{2'}$, where $R^{2'}$ denotes hydrogen or $C_1$-$C_4$-alkyl,

$R^3$ represents a cyclic amino group, such as

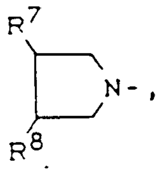
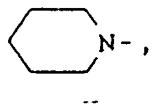
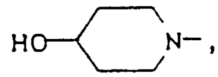
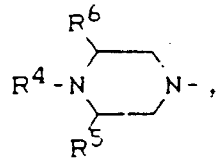

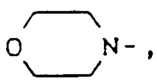
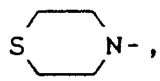
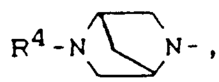
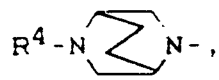

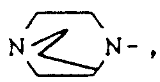
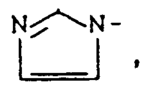
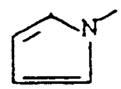

wherein

$R^4$ represents hydrogen, alkyl with 1 to 4 carbon atoms, 2-hydroxyethyl or 2-oxopropyl,

$R^5$ represents hydrogen or methyl,

$R^6$ represents hydrogen or alkyl with 1 to 4 carbon atoms,

$R^7$ represents hydrogen, amino, methylamino, ethylamino, aminomethyl, methylaminomethyl, ethylaminomethyl, dimethylaminomethyl, hydroxyl or hydroxymethyl,

$R^8$ represents hydrogen, methyl, ethyl or chlorine,

X represents halogen, in particular fluorine and chlorine, and

A represents C-$R^{11}$, wherein $R^{11}$ represents hydrogen or halogen,

characterised in that compounds of the formula (II)

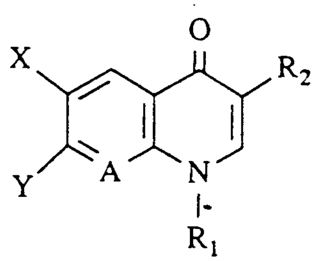

wherein

$R^1$, $R^2$, A and X have the abovementioned meaning, and

Y represents halogen, in particular fluorine and chlorine,

are reacted with solvent-free amines of the formulae

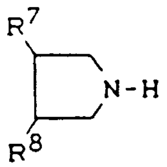, 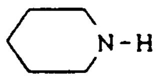, 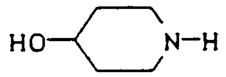, 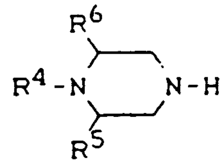,

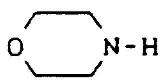, 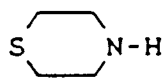, 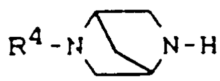, 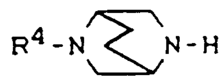,

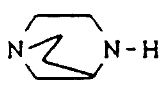, 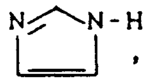, 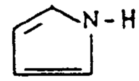

wherein

$R^4$, $R^5$, $R^6$, $R^7$, $R^8$ and $R^{11}$ have the abovementioned meaning,

dispensing with the use of solvents, in a ratio of 1:2 to 1:10 at temperatures between 20 ° C and 200 ° C.

**2.** Process according to Claim 1, characterised in that 1-cyclopropyl-7-chloro-6-fluoro-1,4-dihydro-4-oxo-3-quinolinecarboxylic acid is reacted with piperazine without a solvent at 120 ° to 160 ° C.

**3.** Process according to Claims 1 and 2, characterised in that 1-cyclopropyl-6,7-difluoro-1,4-dihydro-4-oxo-3-quinolinecarboxylic acid is reacted with piperazine without a solvent at 120 ° C to 160 ° C.

**4.** Process according to Claims 1 and 2, characterised in that 1-cyclopropyl-7-chloro-6-fluoro-1,4-dihydro-4-oxo-3-quinolinecarboxylic acid is metered into piperazine which has been heated to 140 ° C to 150 ° C, the mixture is cooled, water is added and the reaction product is crystallised out.

**5.** Process according to Claim 4, characterised in that 1-cyclopropyl-6,7-difluoro-1,4-dihydro-4-oxo-3-quinolinecarboxylic acid is used as the starting substance.

**6.** Process according to Claim 1, characterised in that 1-cyclopropyl-7-chloro-6-fluoro-1,4-dihydro-4-oxo-3-quinolinecarboxylic acid is reacted with 1-ethylpiperazine, 2-hydroxyethylpiperazine or 1-methylpiperazine.

## Revendications

**1.** Procédé pour la fabrication de composés de formule (I)

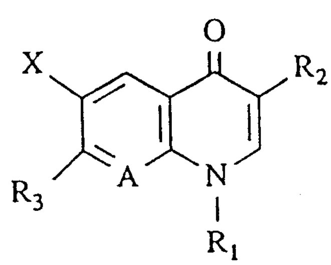

(I)

dans laquelle

$R^1$ représente un groupe méthyle, éthyle, propyle, isopropyle, cyclopropyle, 2-hydroxyéthyle, 2-fluoroéthyle, 4-fluorophényle ou 2,4-difluorophényle,

$R^2$ représente un groupe CN ou $COOR^{2'}$ dans lequel $R^{2'}$ représente l'hydrogène ou un groupe

alkyle en $C_1$-$C_4$,

$R^3$ représente un groupe amino cyclique tel que

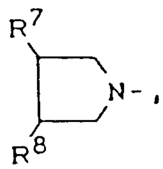 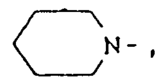 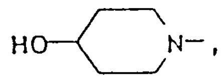 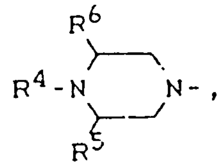

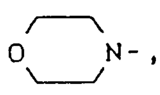 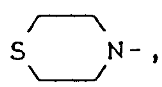 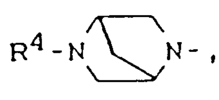 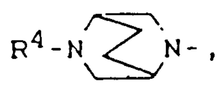

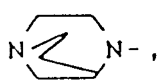 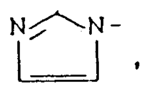 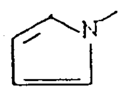

dans lesquels

$R^4$ représente un atome d'hydrogène ou un groupe alkyle en $C_1$-$C_4$,2-hydroxyéthyle ou 2-oxopropyle,

$R^5$ représente un atome d'hydrogène ou un groupe méthyle,

$R^6$ représentte un atome d'hydrogène ou un groupe alkyle en $C_1$-$C_4$,

$R^7$ représente un atome d'hydrogène ou un groupe amino, méthylamino, éthylamino, aminométhyle, méthylaminoéthyle, éthylaminométhyle, diméthylaminométhyle, hydroxy ou hydroxyméthyle,

$R^8$ représente un atome d'hydrogène ou de chlore ou un groupe méthyle ou éthyle,

X représente un atome d'halogène, en particulier fluor ou chlore et

A représente $\geqslant$C-$R^{11}$ ou $R^{11}$ représente un atome d'hydrogène ou d'halogène,

caractérisé en ce que l'on fait réagir des composés de formule (II)

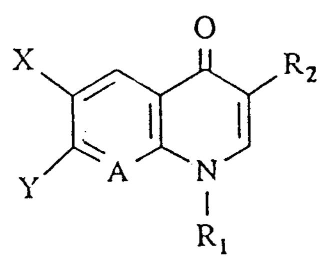

(II)

dans laquelle

$R^1$, $R^2$, A et X ont les significations indiquées ci-dessus, et

Y représente un halogène, en particulier le fluor ou le chlore,

avec des amines de formules suivantes exemptes de solvant

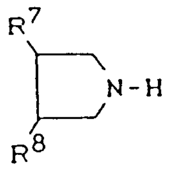, 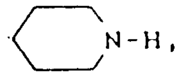, 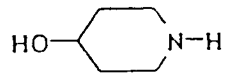, 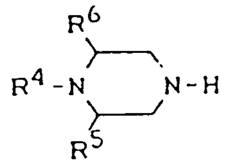,

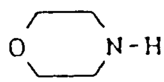, 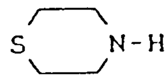, 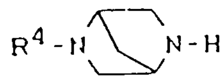, 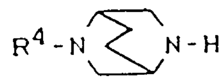,

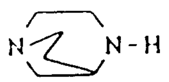, 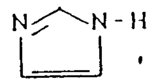, 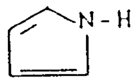

dans lesquels

$R^4$, $R^5$, $R^6$, $R^7$, $R^8$ et $R^{11}$ ont les significations indiquées ci-dessus

avec les amines pures dans le rapport de 1:2 à 1:10 à des températures comprises entre 20 et 200 ° C en renonçant à l'application de solvants.

2. Procédé selon la revendication 1, caractérisé en ce que l'on fait réagir l'acide 1-cyclopropyl-7-chloro-6-fluoro-1,4-dihydro-4-oxo-3-quinoléine-carboxylique avec la pipérazine sans solvant à 120-160 ° C.

3. Procédé selon les revendications 1 et 2, caractérisé en ce que l'on fait réagir l'acide 1-cyclopropyl-6,7-difluoro-1,4-dihrdro-4-oxo-3-quinoléine-carboxylique avec la pipérazine sans solvant à 120-160 ° C.

4. Procédé selon les revendications 1 et 2, caractérisé en ce que l'on dose l'acide 1-cyclopropyl-7-chloro-6-fluoro-1,4-dihydro-4-oxo-3-quinoléine-carboxylique dans la pipérazine, qui a été chauffée à 140-150 ° C, on refroidit, on ajoute de l'eau et on sépare le produit de réaction cristallisé.

5. Procédé selon la revendication 4, caractérisé en ce que l'on utilise comme produit de départ l'acide 1-cyclopropyl-6,7-difluoro-1,4-dihydro-4-oxo-3-quinoléine-carboxylique.

6. Procédé selon la revendication 1, caractérisé en ce que l'on fait réagir l'acide 1-cyclopropyl-7-chloro-6-fluoro-1,4-dihydro-4-oxo-3-quinoléine-carboxylique avec la 1-éthylpipérazine, la 2-hydroxyéthylpipérazine ou la 1-méthylpipérazine.